# EUROPEAN PATENT APPLICATION

(11) **EP 3 428 645 A1**
(43) Date of publication of application: **16.01.2019**
(21) Application number: 17763314.6
(22) Date of filing: 08.03.2017
(51) Int. Cl.: G01N 33/483, G01N 33/50

(54) **SIMPLE SPERM TEST KIT, SYSTEM, AND METHOD FOR PERFORMING SIMPLE TEST ON SPERM**

(30) Priority: 08.03.2016 JP 2016044615
(71) Applicant: Recruit Co., Ltd., Chuo-ku Tokyo 1040061 (JP)
(72) Inventor: IRISAWA, Ryo, Tokyo 100-6640 (JP)
(74) Representative: EIP
(86) International application number: PCT/JP2017/009213
(87) International publication number: WO 2017/154974

(57) **Abstract**

A simple sperm test kit 10 for performing a simple sperm test has a substrate 20 capable of being placed over a camera 30 of an information terminal 11, a recess 21 for containing semen A provided in the surface of the substrate 20, a cover 22 that covers the recess 21 for allowing external light to enter the recess 21, and a lens 23 provided within the substrate 20 on the lower side of the recess 21 for magnifying the semen A in the recess 21 and projecting an image on the back side of the substrate 20.

## Description

### Cross-Reference to Related Applications

The present application is based on Japanese Patent Application No. 2016-044615 filed on March 8, 2016, the contents of which are incorporated herein by reference.

### Technical Field

The present application relates to a simple sperm test, simple sperm test system, and simple sperm test method.

### Background Art

Male sperm abnormalities are considered to be one of the causes of infertility. A specialized medical institution is typically required to test whether or not sperm is normal (see Patent Document 1).

### Citation List

### Patent Document

Patent Document 1: Patent Publication JP-A-2009-288189

### Summary

### Technical Problem

However, in the case of such sperm tests as described above, there is the considerable burden of having to collect a semen sample at a hospital or other medical institution or submit a collected semen sample to a hospital or the like. In addition, both time and costs are required until test results are obtained. As a result, there are many cases in which such tests are unable to be performed readily at medical institutions thereby resulting in a delay in the detection of sperm abnormalities. It would therefore be desirable if it were possible to easily perform this test at home or other convenient location prior to having the test performed at health care institution.

With the foregoing in view, an object of the present invention is to enable a simple sperm test to be performed easily at home or other convenient location.

### Solution to Problem

As a result of conducting extensive studies, the inventors of the present invention found the above-mentioned problems can be solved by using a simple sperm test kit to capture a video of sperm with an information terminal equipped with a camera, transmitting that video, and receiving the results of the simple sperm test with that information terminal, thereby leading to completion of the present invention.

Namely, the present invention includes the aspects indicated below.
(1) A simple sperm test kit for performing a simple sperm test, having: a single substrate capable of being placed over a camera of an information terminal, a recess for containing semen provided in the surface of the substrate, a cover that covers the recess for allowing external light to enter the recess, and a lens immobilized within the substrate on the lower side of the recess for magnifying the semen in the recess and projecting an image on the back side of the substrate.
(2) The simple sperm test kit described in (1), wherein the cover is integrated with the substrate and a notch is formed in a portion of the cover.
(3) The simple sperm test kit described in (1) or (2), wherein the back side of the cover has been subjected to hydrophilic treatment.
(4) The simple sperm test kit described in any of (1) to (3), wherein spacers are provided in the recess for maintaining a fixed distance between the bottom of the recess and the back side of the cover, and the spacers are provided at locations overlapping with the lens when viewed from overhead.
(5) The simple sperm test kit as described in (4), wherein the height of the spacers is 10 µm to 20 µm.
(6) The simple sperm test kit described in any of (1) to (5), wherein depressions are formed in the surface of the substrate on both the left and right sides of the recess and the cover is supported by the depressions.
(7) A system for performing a simple sperm test, having: an information terminal equipped with a camera having a video filming function and a simple sperm test kit; wherein, the simple sperm test kit has a single substrate capable of being placed over a camera of the information terminal, a recess for containing semen provided in the surface of the substrate, a cover that covers the recess for allowing external light to enter the recess, and a lens immobilized within the substrate on the lower side of the recess for magnifying the semen in the recess and projecting an image on the back side of the substrate, and the information terminal has a filming control unit for filming a video of a magnified image of the semen with a camera, a video display control unit for displaying the video of the magnified image of the semen on a display screen, a communication unit for transmitting the video to an external server and receiving the results of the simple sperm test from the external server, and a test results display control unit for displaying the results of the simple sperm test on the display screen.
(8) The system described in (7), wherein the substrate of the simple sperm test kit can be placed over a camera provided on the same side as the display screen of the information terminal.
(9) The system described in (7) or (8), wherein the cover is integrated with the substrate and a notch is formed in a portion of the cover.
(10) The system described in any of (7) to (9), wherein the back side of the cover has been subjected to hydrophilic treatment.
(11) The system described in any of (7) to (10), wherein spacers are provided in the recess for maintaining a fixed distance between the bottom of the recess and the back side of the cover, and the spacers are provided at locations overlapping with the lens when viewed from overhead.
(12) The system described in (11), wherein the height of the spacers is 10 µm to 20 µm.
(13) The system described in any of (7) to (12), wherein depressions are formed in the surface of the substrate on both the left and right sides of the recess and the cover is supported by the depressions.
(14) A method for carrying out a simple sperm test, which comprises the steps of: placing a simple sperm test kit over a camera of an information terminal equipped with a camera having a video filming function, the simple sperm test kit having a single substrate, a recess for containing semen provided in the surface of the substrate, a cover that covers the recess for allowing external light to enter the recess, and a lens immobilized within the substrate on the lower side of the recess for magnifying the semen in the recess and projecting an image on the back side of the substrate, and filming a video of a magnified image of the semen with the camera; displaying the video of the magnified image of the semen on a display screen of the information terminal; transmitting the video from the communication unit of the information terminal to an external server; receiving the results of the simple sperm test from the external server; and displaying the results of the simple sperm test on the display screen of the information terminal.
(15) The method for carrying out a simple sperm test described in (14), wherein the simple sperm test kit is placed over a camera provided on the same side as the display screen of the information terminal and the video of the magnified image of the semen is filmed while displaying on the display screen. Advantageous Effects of Invention

According to the present invention, a simple sperm test can be easily carried out at home.

### Brief Description of Drawings

Fig. 1 is an explanatory drawing showing an example of the configuration of a simple sperm test system.
Fig. 2 is a cross-sectional view showing an example of the configuration of a simple sperm test kit.
Fig. 3 is an enlarged view of the surface of a simple sperm test kit.
Fig. 4 is a block diagram showing the functions of an information terminal.
Fig. 5 is an explanatory drawing depicting the display of a video of semen on the display screen of an information terminal.
Fig. 6 is an explanatory drawing depicting the transmission of a video of semen on the display screen of an information terminal.
Fig. 7 is an explanatory drawing depicting the display of the results of a simple sperm test on the display screen of an information terminal.
Fig. 8 is a cross-sectional drawing taken along line Y-Y of Fig. 9 showing an example of another configuration of a simple sperm test kit.
Fig. 9 is an enlarged view of the surface of a simple sperm test kit employing another example of a configuration.

### Description of Embodiments

The following provides an explanation of preferred embodiments of the present invention with reference to the drawings. Furthermore, the same reference signs are used to indicate the same elements and duplicate explanations are omitted. In addition, positional relationships such as the up, down, left and right directions are based on the positional relationships indicated in the drawings unless specifically indicated otherwise. Moreover, size ratios in the drawings are not limited to the ratios shown in the drawings. Moreover, the following embodiments are provided as examples for explaining the present invention and the present invention is not limited to these embodiments.

### <Simple Sperm Test System>

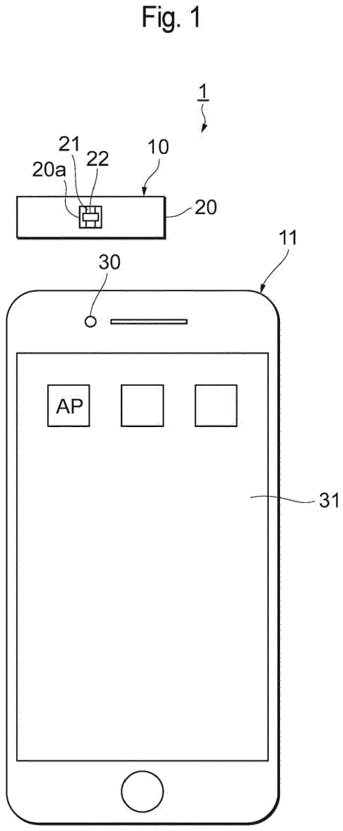

As shown in Fig. 1, a simple sperm test system 1 has a simple sperm test kit 10 and an information terminal 11 equipped with a camera having a video filming function.

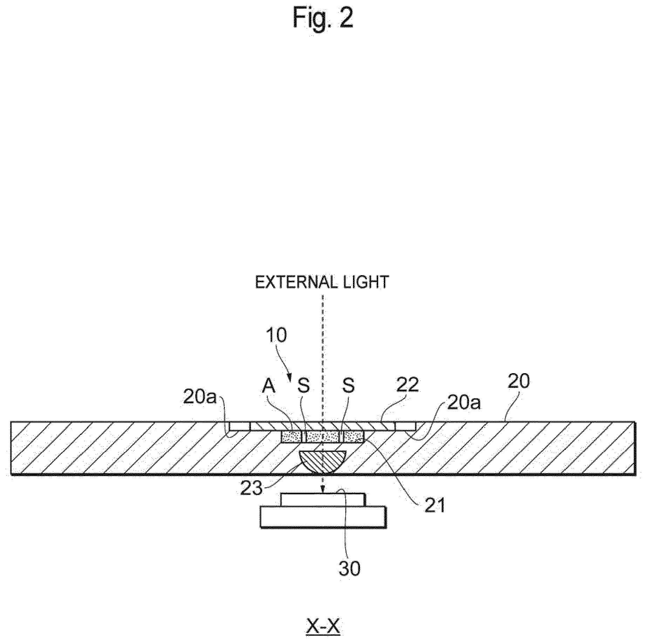

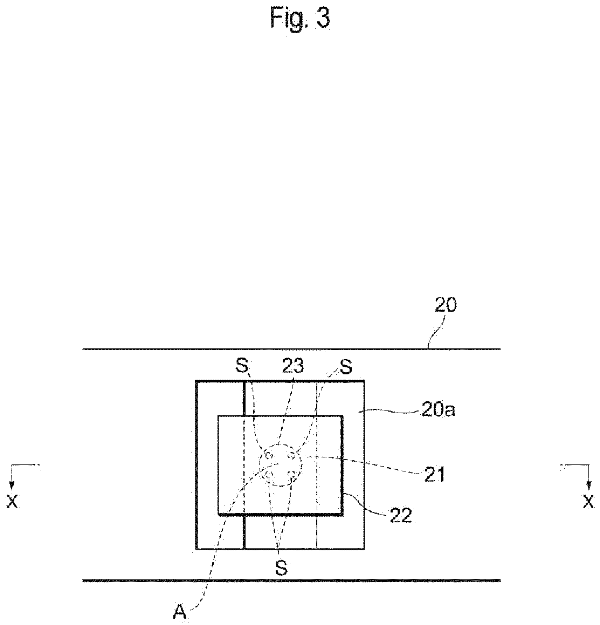

For example, as shown in Fig. 2, the simple sperm test kit 10 has a substrate 20 that can be placed over a camera 30 of the information terminal 11. The substrate 20 is made of plastic, for example, and has a thin, rectangular shape. A recess 21 for containing semen A is formed in the center of the surface of the substrate 20 as shown in Figs. 2 and 3. The recess 21 is formed, for example, into a rectangular shape that is longer in the direction perpendicular to the lengthwise direction of the substrate 20 when viewed from overhead. The depth of the recess 21 (distance between the lower side of a cover 22 and the bottom of the recess 21) is set to 10 µm or more, preferably 10 µm to 20 µm, and more preferably 10 µm, in consideration of the thickness of the head of a typical sperm. Furthermore, the shape of the recess 21 is not limited to a rectangular shape, but rather may also have a circular or other shape.

The rectangular cover 22 that covers the recess 21 can be placed on the surface of the substrate 20. For example, a rectangular depression 20a that is larger than the recess 21 is formed on the surface of the substrate 20, and the cover 22 can be placed on the depression 20a. The cover 22 is made of clear plastic and allows external light to pass there through and enter the recess 21. At least the back side of the cover 22 is subjected to, for example, hydrophilic treatment. Furthermore, the cover 22 may cover a portion of the recess 21 or cover the entire recess 21.

Spacers S are provided in the recess 21 for maintaining a fixed distance between the bottom of the recess 21 and the back side of the cover 22. The spacers S are formed into the shape of, for example, narrow cylindrical pins and are erected on the bottom of the recess 21. A plurality of the spacers S, such as 4 as shown in Fig. 3, are provided and arranged circumferentially at equal intervals centered on the center of the recess 21 when viewed from overhead.

A hemispherical lens 23 is provided in the substrate 20 on the lower side in the center of the recess 21 that magnifies the semen A present in the recess 21 and projects an image thereof on the back side of the substrate 20 as shown in Fig. 2. The hemispherical lens 23 is made of plastic, for example. Furthermore, the lens is not limited to the hemispherical lens 23, but rather may also be a ball or other type of lens.

Furthermore, the substrate 20, the cover 22 and the hemispherical lens 23 may all be formed with the same material such as acrylic.

The information terminal 11 is, for example, a smartphone or tablet equipped with the camera 30 having a video filming function as shown in Fig. 1. The information terminal 11 has the camera 30 on the same side as the display screen 31, for example.

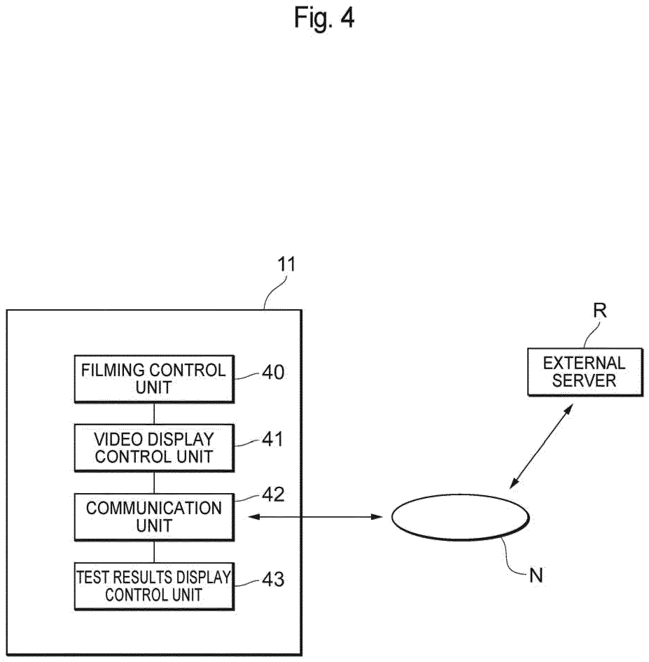

As shown in Fig. 4, the information terminal 11 has a filming control unit 40 that films a video of a magnified image of the semen A with the camera 30, a video display control unit 41 that display a video of a magnified image of the semen A on the display screen 31, a communication unit 42 that transmits the video to an external server R via a communication network N such as the Internet and receives the results of a simple sperm test from the external server R, and a test results display control unit 43 that displays the results of the simple sperm test on the display screen 31. The information terminal 11 has an application (program) AP that is launched by the information terminal 11 and is able to execute the simple sperm test method to be subsequently described by using the functions of the above-mentioned filming control unit 40, the video display control unit 41, the communication unit 42 and the test results display control unit 43. This application AP can be downloaded to the information terminal 11 via the communication network N.

### <Simple Sperm Test Method>

The following provides an explanation of a simple sperm test method using the above-mentioned simple sperm test system 1.

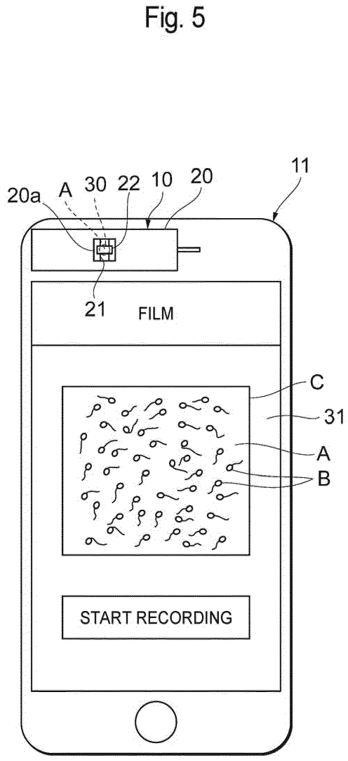

First, a drop of the semen A is placed with a dropper in the vicinity of the hemispherical lens 23 in the center of the recess 21 in the substrate 20 of the simple sperm test kit 10 followed by covering the sperm with the cover 22 (Fig. 2). At this time, the distance between the back side of the cover 22 and the bottom of the recess 21 is maintained by spacers S at a fixed distance that is preferable for ensuring an area of activity for the sperm. Next, the simple sperm test kit 10 is placed over the camera 30 of the information terminal 11 on the same side as the display screen 31 as shown in Fig. 5.

Next, the application AP is launched by the information terminal 11. As a result, the camera 30 of the information terminal 11 is activated and video filming C is started by the filming control unit 40. At this time, in the simple sperm test kit 10, external light enters the recess 21 from the cover 22, passes through the semen A and then passes through hemispherical lens 23 as shown in Fig. 2. As a result, an image of the semen A (sperm B) within the recess 21 is magnified by the hemispherical lens 23 and projected and filmed by the camera 30. The video C filmed by the camera 30 is displayed on the display screen 31 of the information terminal 11 by the video display control unit 41 as shown in Fig. 5. The video C of the magnified semen A (sperm B) is thus displayed on the display screen 31 in real time. At this time, if the location of the simple sperm test kit 10 is out of position, the location thereof is adjusted while viewing the display screen 31.

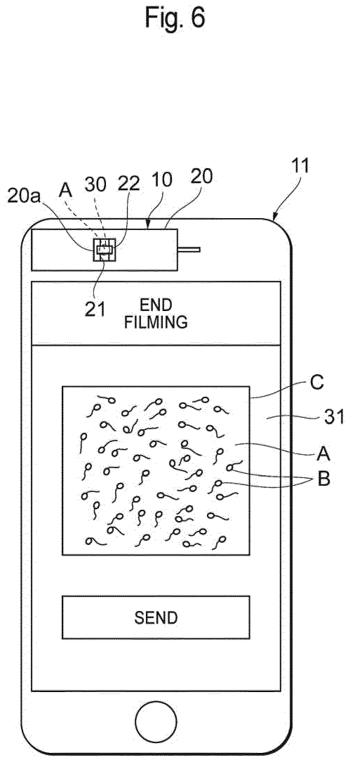

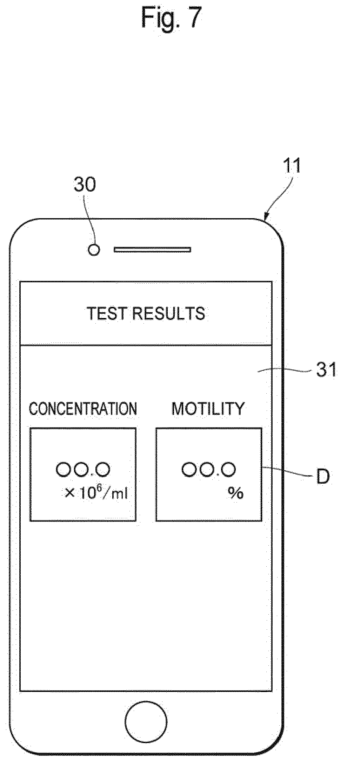

Next, the video C of the semen A displayed on the display screen 31, for example, is recorded for a prescribed amount of time by pressing a record button. Subsequently, as shown in Fig. 6, the video C is transmitted to the external server R via the communication network N by the communication unit 42 by pressing a send button. A simple sperm test is carried out in the external server R based on the received video data, for example. For example, video data is analyzed and sperm concentration and motility are calculated as the results of the simple sperm test. Subsequently, the results of the simple sperm test are transmitted to the information terminal 11 by the external server R and received with the communication unit 42 of the information terminal 11. Subsequently, results D of the simple sperm test are displayed on the display screen 31 of the information terminal 11 by the test results display control unit 43 as shown in Fig. 7. Furthermore, the results of the simple sperm test may also include parameters such as motile sperm count, straight-moving sperm count, sperm velocity or normal morphology rate.

According to the present embodiment, a simple sperm test can be easily performed at home or other convenient location by filming a video of sperm by placing the simple sperm test kit 10 over the camera 30 of the information terminal 11, sending that video from the information terminal 11 to the external server R, and receiving the results of the simple sperm test from the external server R.

The simple sperm test kit 10 has the substrate 20 capable of being placed over the camera 30 of the information terminal 11, the recess 21 for containing the semen A, the cover 22 that allows external light to enter the recess 21, and the hemispherical lens 23 that magnifies the semen A in the recess 21 and projects that image on the back side of the substrate 20. As a result, a magnified video of the semen A can be filmed simply by placing the semen A in the recess 21 of the substrate 20, covering the recess 21 with the cover 22, and placing the substrate 20 over the camera 30 of the information terminal 11. Accordingly, a simple sperm test can be easily realized. Since external light can be used for the light source, the configuration of the simple sperm test kit 10 can be simplified and provided inexpensively and in a compact form.

Since the simple sperm test kit 10 can be placed over the camera 30 on the side of the display screen 31 of the information terminal 11, the location of the simple sperm test kit 10 can be adjusted while viewing the display screen 31.

Since the surface of the cover 22 is subjected to hydrophilic treatment, the semen A is able to reliably spread out within the recess 21 even if only a slight amount thereof is present. As a result, a magnified image of the semen A can be suitably acquired.

Since spacers S are provided in the recess 21, a fixed distance is maintained between the bottom of the recess 21 and the back side of the cover 22, and since the cover 22 is not deflected, the inside of the recess 21 is maintained at the optimal depth for filming while ensuring an area of activity for the sperm.

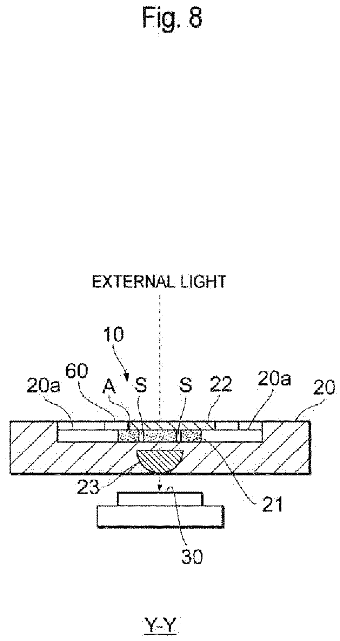

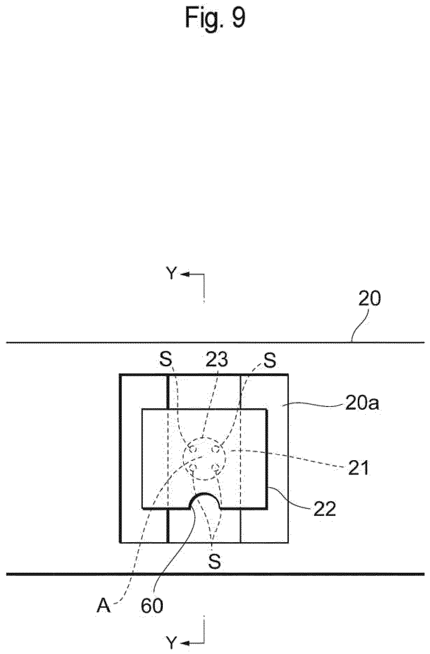

Although the cover 22 is separate from the substrate 20 in the above-mentioned embodiment, the cover 22 may also be integrated into a single unit with the substrate 20. In this case, for example, a notch 60 that leads to the recess 21 is formed in a portion of the cover 22 as shown in Fig. 8 and Fig. 9. The notch 60 is formed, for example, in the shape of an arc in the center of a side of the rectangular cover 22. Furthermore, the location and shape of the notch 60 can be selected arbitrarily, and for example, the shape of the notch 60 may be rectangular, triangular or circular. The back side of the cover 22 is subjected to hydrophilic treatment. Furthermore, the term "integrated" may refer to being integrally molded or refer to being molded separately and then mutually adhered.

When performing the simple sperm test, the semen A is placed in the recess 21 through the notch 60 of the cover 22 using a dropper and the like. The semen A spreads throughout the recess 21 due to the hydrophilicity of the cover 22. Subsequently, a simple sperm test can be performed by placing the simple sperm test kit 10 over the camera 30, filming a video of the semen A, transmitting that video to the external server R and receiving the results of the simple sperm test from the external server R in the same manner as the above-mentioned embodiment.

According to the present embodiment, since the cover 22 is integrated with the substrate 20, the simple sperm test kit 10 is only composed of a single part, thereby resulting in easier handling and enabling the simple sperm test to be performed more easily.

Although the above has provided an explanation of preferred embodiments of the present invention with reference to the drawings, the present invention is not limited thereto. A person with ordinary skill in the art would clearly be able to conceive of various examples of alterations and modifications within the scope of the idea described in the claims, and such alterations and modifications are naturally also understood to belong to the technical scope of the present invention.

For example, there are no particular limitations on the size, shape or material of the simple sperm test kit 10 in the above-mentioned embodiments. The functions of the information terminal 11 are not limited to those indicated in the above-mentioned examples.

### Industrial Applicability

The present invention is useful for easily performing a simple sperm test at home or other convenient location.

### Reference Signs List

- 1: Simple sperm test system
- 10: Simple sperm test kit
- 11: Information terminal
- 20: Substrate
- 21: Recess
- 22: Cover
- 23: Hemispherical lens
- 30: Camera
- A: Semen
- B: Sperm

## Claims

1. A simple sperm test kit for performing a simple sperm test, comprising:
a single substrate capable of being placed over a camera of an information terminal,
a recess for containing semen provided in the surface of the substrate,
a cover that covers the recess for allowing external light to enter the recess, and
a lens immobilized within the substrate on the lower side of the recess for magnifying the semen in the recess and projecting an image on the back side of the substrate.

2. The simple sperm test kit according to claim 1, wherein the cover is integrated with the substrate, and
a notch is formed in a portion of the cover.

3. The simple sperm test kit according to claim 1 or 2, wherein the back side of the cover has been subjected to hydrophilic treatment.

4. The simple sperm test kit according to claim 1 or 2, wherein spacers are provided in the recess for maintaining a fixed distance between the bottom of the recess and the back side of the cover, and
the spacers are provided at locations overlapping with the lens when viewed from overhead.

5. The simple sperm test kit according to claim 4, wherein the height of the spacers is 10 µm to 20 µm.

6. The simple sperm test kit according to claim 1 or 2, wherein depressions are formed in the surface of the substrate on both the left and right sides of the recess, and
the cover is supported by the depressions.

7. A system for performing a simple sperm test, comprising:
an information terminal equipped with a camera having a video filming function, and
a simple sperm test kit; wherein,
the simple sperm test kit has a single substrate capable of being placed over a camera of the information terminal,
a recess for containing semen provided in the surface of the substrate,
a cover that covers the recess for allowing external light to enter the recess, and
a lens immobilized within the substrate on the lower side of the recess for magnifying the semen in the recess and projecting an image on the back side of the substrate; and,
the information terminal has:
a filming control unit for filming a video of a magnified image of the semen with a camera,
a video display control unit for displaying a video of the magnified image of the semen on a display screen,
a communication unit for transmitting the video to an external server and receiving the results of the simple sperm test from the external server, and
a test results display control unit for displaying the results of the simple sperm test on the display screen.

8. The system according to claim 7, wherein the substrate of the simple sperm test kit can be placed over a camera provided on the same side as the display screen of the information terminal.

9. The system according to claim 7 or 8, wherein the cover is integrated with the substrate, and
a notch is formed in a portion of the cover.

10. The system according to claim 7 or 8, wherein the back side of the cover has been subjected to hydrophilic treatment.

11. The system according to claim 7 or 8, wherein spacers are provided in the recess for maintaining a fixed distance between the bottom of the recess and the back side of the cover, and
the spacers are provided at locations overlapping with the lens when viewed from overhead.

12. The system according to claim 11, wherein the height of the spacers is 10 µm to 20 µm.

13. The system according to claim 7 or 8, wherein depressions are formed in the surface of the substrate on both the left and right sides of the recess, and
the cover is supported by the depressions.

14. A method for carrying out a simple sperm test, which comprises the steps of:
placing a simple sperm test kit over a camera of an information terminal equipped with a camera having a video filming function, the simple sperm test kit having a single substrate, a recess for containing semen provided in the surface of the substrate, a cover that covers the recess for allowing external light to enter the recess, and a lens immobilized within the substrate on the lower side of the recess for magnifying the semen in the recess and projecting an image on the back side of the substrate, and filming a video of a magnified image of the semen with the camera;
displaying the video of the magnified image of the semen on a display screen;
transmitting the video from the communication unit of the information terminal to an external server;
receiving the results of the simple sperm test from the external server; and
displaying the results of the simple sperm test on the display screen of the information terminal.

15. The method for carrying out a simple sperm test according to claim 14, wherein the simple sperm test kit is placed over a camera provided on the same side as the display screen of the information terminal and the video of the magnified image of the semen is filmed while displaying on the display screen.
